Europäisches Patentamt

European Patent Office

Office européen des brevets

(19)

(11) Veröffentlichungsnummer: **0 261 549**
**A1**

## (12) EUROPÄISCHE PATENTANMELDUNG

(21) Anmeldenummer: 87113434.2

(22) Anmeldetag: 15.09.87

(51) Int. Cl.⁴: **C07C 67/54** , C07D 213/16

(30) Priorität: 18.09.86 DE 3631679

(43) Veröffentlichungstag der Anmeldung:
30.03.88 Patentblatt 88/13

(84) Benannte Vertragsstaaten:
**BE DE ES FR GB IT NL**

(71) Anmelder: **BASF Aktiengesellschaft**
**Carl-Bosch-Strasse 38**
**D-6700 Ludwigshafen(DE)**

(72) Erfinder: **Leman, Otto**
**Im Wandelgarten 8**
**D-6719 Weisenheim(DE)**
Erfinder: **Lendle, Hubert, Dr.**
**Ruedigertrasse 41**
**D-6700 Ludwigshafen(DE)**
Erfinder: **Magnussen, Peter, Dr.**
**Professor-Dillinger-Weg 25**
**D-6702 Bad Duerkheim(DE)**

(54) Verfahren zur Abtrennung von Pyridin aus Gemischen von Pyridin und Isovaleriansäuremethylester.

(57) Verfahren zur Abtrennung von Pyridin aus Gemischen von Pyridin und Isovaleriansäuremethylester, dadurch gekennzeichnet, daß man das Gemisch aus Pyridin und Isovaleriansäuremethylester unter Zusatz von Wasser einer Destillation unterwirft, Isovaleriansäuremethylester als Azeotrop mit Wasser abtrennt und Pyridin als Sumpfprodukt gewinnt.

EP 0 261 549 A1

# Verfahren zur Abtrennung von Pyridin aus Gemischen von Pyridin und Isovaleriansäuremethylester

Aus der DE-OS 16 18 156 ist die Herstellung von Isovaleriansäureestern durch Umsetzen von Isobutylen mit Alkanolen und Kohlenmonoxid in Gegenwart von Cobaltcarbonylkatalysatoren und Pyridin bekannt. Es hat sich nun herausgestellt, daß sich bei der Herstellung Isovaleriansäuremethylester, die hierbei anfallenden Gemische aus Pyridin und Isovaleriansäuremethylester durch Destillation nicht trennen lassen.

Es war deshalb die technische Aufgabe gestellt, einen Weg zu finden, Pyridin aus Gemischen von Pyridin und Isovaleriansäuremethylester auf einfache Weise abzutrennen.

Diese Aufgabe wird gelöst in einem Verfahren zur Abtrennung von Pyridin aus Gemischen von Pyridin und Isovaleriansäuremethylester, wobei man das Gemisch aus Pyridin und Isovaleriansäuremethylester unter Zusatz von Wasser einer Destillation unterwirft, Isovaleriansäuremethylester als Azetrop mit Wasser abdestilliert und Pyridin als Sumpfprodukt gewinnt.

Das neue Verfahren hat den Vorteil, daß es auf einfache Weise gelingt, Pyridin von Isovaleriansäuremethylester abzutrennen. Ferner hat das neue Verfahren den Vorteil, daß es gelingt, Pyridin wasserfrei wiederzugewinnen.

Erfindungsgemäß geht man von Gemischen aus Pyridin und Isovaleriansäuremethylester aus. Solche Gemische enthalten in der Regel 80 bis 95 Gew.-% Pyridin und 2 bis 12 Gew.-% Isovaleriansäuremethylester. Daneben können sie Verunreinigungen wie Valeriansäuremethylester, Methanol, Cyclohexan, z.B. in Mengen bis zu 18 Gew.-% enthalten. Ein typisches Gemisch enthält beispielsweise 90 bis 94 Gew.-% Pyridin 3 bis 4 Gew.-% Isovaleriansäuremethylester und 2 bis 7 Gew.-% Verunreinigungen. Solche Gemische erhält man beispielsweise bei der Carbalkoxylierung von Isobutylen, Buten oder $C_4$-Schnitten wie sie beispielsweise in der DE-OS 16 18 156 beschrieben wird.

Entsprechend dem erfindungsgemäßen Verfahren wird das Gemisch aus Pyridin und Isovaleriansäuremethylester unter Zusatsz von Wasser einer Destillation unterworfen. Isovaleriansäuremethylester wird hierbei als Azeotrop mit Wasser abdestilliert. Das hierbei gebildete Azeotrop besteht aus 77,7 Gew.-% Isovaleriansäuremethylester und 22,3 Gew.-% Wasser und hat einen Siedepunkt von 88,5°C. Pyridin wird bei der Destillation als Sumpf produkt gewonnen. Vorteilhaft setzt man dem Ausgangsgemisch mindestens soviel Wasser zu wie zur Bildung des Azeotrops Isovaleriansäuremethylester/Wasser erforderlich ist, insbesondere die 1-bis 3-fache Menge. Pyridin fällt dann wasserfrei im Sumpf an.

Vorteilhaft wird die Destillation in einer üblichen Kolonne, z.B. Glockenbodenkolonne, Füllkörperkolonne, Siebbodenkolonne oder in einer geordnet gepackten Kolonne durchgeführt, die zweckmäßig 20 bis 40 theoretische Böden hat. Das Ausgangsgemisch Pyridin und Isovaleriansäure wird unter Zusatz der erforderlichen Menge Wasser, vorzugsweise dem mittleren Teil der Kolonne zugeführt und als Kopfprodukt das Azeotrop Isovaleriansäure/Wasser abgezogen, während man bei Einhaltung der optimalen Wassermenge als Sumpfprodukt wasserfreies Pyridin gewinnt. Es hat sich auch bewährt, wenn man ein Rücklaufverhältnis von 30 bis 70 : 1 einhält.

Nach dem Verfahren der Erfindung erhält man Isovaleriansäuremethylester zur Verwendung beispielsweise als Lösungsmittel, während wasserfreies Pyridin wiederum für die Carbalkoxylierung wiederverwendbar ist.

Das Verfahren nach der Erfindung sei an folgendem Beispiel veranschaulicht.

## Beispiel

Einer Glockenbodenkolonne mit 30 praktischen Böden wird ein auf 73°C vorgeheiztes Gemisch folgender Zusammensetzung auf den 12. praktischen Boden zugeführt: 86,9 % (m/m) Pyridin, 3,3 % (m/m) i-Valeriansäuremethylester, (iVSE) 5,2 % (m/m) Valeriansäuremethylester (VSE), 1,4 % (m/m) Cyclohexan, 2,4 % (m/m) Wasser und 0,8 % (m/m) Unbekannte. Bei einem Rücklaufverhältnis von 40 : 1, es werden beide Phasen des Heteroazeotrops wie sie anfallen zurückgeführt, erhält man folgende Zusammensetzung des Sumpfablaufs: 95,8 % (m/m) Pyridin, 0,3 % (m/m) iVSE, 3,4 % (m/m) VSE, 0,5 % Unbekannte und 0,06 % Wasser. Das Kopfprodukt fällt zweiphasig an; auf 1 Gewichtsteil schwere Phase entfallen 3,25 Gewichtsteile leichte Phase. Die Zusammensetzung der schweren Destillat-Phase lautet: 0,2 % (m/m) Cyclohexan, 10 % (m/m) Pyridin, 0,1 % (m/m) iVSE, 0,5 % (m/m) Unbekannte und 89,2 % (m/m) Wasser. Die Analyse der leichten Phase zeigt 17,2 % (m/m) Cyclohexan, 15 % (m/m) Pyridin, 36,5 % (m/m) iVSE, 25,1 % (m/m) VSE, 4,5 % (m/m) Unbekannte und 1,7 % (m/m) Wasser.

Bei der Destillation wird 10,7 % (m/m) des Zulaufs über Kopf genommen. Das im Kopfprodukt enthaltene Pyridin kann nach Extraktion mit einem Wasser-Essigsäuregemisch und Kreisführung des Extraktes vollständig wiedergewonnen werden. Die Kopftemperatur beträgt 82°C, die Sumpftemperatur beträgt 116°C.

**Ansprüche**

1. Verfahren zur Abtrennung von Pyridin aus Gemischen von Pyridin und Isovaleriansäuremethylester, dadurch gekennzeichnet, daß man das Gemisch aus Pyridin und Isovaleriansäuremethylester unter Zusatz von Wasser einer Destillation unterwirft, Isovaleriansäuremethylester als Azeotrop mit Wasser abtrennt und Pyridin als Sumpfprodukt gewinnt.

2. Verfahren nach Anspruch 1, dadurch gekennzeichnet, daß man die Menge des zugesetzten Wassers so bemißt, daß sie mindestens der für die Bildung des Azeotrops Isovaleriansäuremethylester/Wasser erforderlichen Menge entspricht und wasserfreies Pyridin als Sumpfprodukt gewinnt.

3. Verfahren nach den Ansprüchen 1 und 2, dadurch gekennzeichnet, daß man das Gemisch aus Pyridin und Isovaleriansäuremethylester sowie Wasser dem mittleren Teil einer Kolonne zuführt und als Azeotrop Isovaleriansäuremethylester/Wasser als Kopfprodukt entnimmt und als Sumpfprodukt Pyridin gewinnt.

**Europäisches Patentamt**

# EUROPÄISCHER RECHERCHENBERICHT

Nummer der Anmeldung

## EINSCHLÄGIGE DOKUMENTE

EP 87113434.2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 047 830 (CHEM. WERKE HÜLS) <br><br> * Zusammenfassung * <br><br> -- | 1 | C 07 C 67/54 <br><br> C 07 D 213/16 |
| A | DE - A1 - 3 016 653 (CHEM. WERKE HÜLS) <br><br> * Anspruch 1 * <br><br> -- | 1 | |
| D,A | DE - A - 1 618 156 (BASF) <br><br> -- | | |
| A | DE - B - 1 220 420 (DISTILLERS COMP.) <br><br> ---- | | |

| RECHERCHIERTE SACHGEBIETE (Int. Cl.4) |
|---|
| C 07 C 67/00 <br><br> C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 22-12-1987 | HOCHHAUSER |